# FASCICULE DE BREVET EUROPEEN

(11) **EP 3 749 262 B1**
(45) Date de publication et mention de la délivrance du brevet: **21.05.2025**
(21) Numéro de dépôt: 19705134.5
(22) Date de dépôt: 05.02.2019
(51) Int. Cl.: A61F 9/00, A61M 11/00, A61M 15/00

(54) **DISPOSITIF D'ASSISTANCE À L'UTILISATION D'UN DISPOSITIF DE DISTRIBUTION DE PRODUIT LIQUIDE**
VORRICHTUNG ZUR UNTERSTÜTZUNG DER VERWENDUNG EINER VORRICHTUNG ZUR ABGABE EINES FLÜSSIGEN PRODUKTS
DEVICE FOR ASSISTING WITH THE USE OF A DEVICE FOR DISPENSING A LIQUID PRODUCT

(30) Priorité: 06.02.2018 FR 1850987
(43) Date de publication de la demande: 16.12.2020
(73) Titulaire: NEMERA LA VERPILLIERE, 38290 La Verpillière (FR)
(72) Inventeur: DECOCK, Thierry, 69007 Lyon (FR); CASSAGNE, Loïck, 38080 Saint-Alban-de-Roche (FR); PINTUS, Pierre, 38300 Crachier (FR)
(74) Mandataire: LLR
(86) Numéro de dépôt international: PCT/EP2019/052777
(87) Numéro de publication internationale: WO 2019/154807

(56) Documents cités:
- DE-U1- 202016 003 139
- JP-A- 2005 131 887
- JP-A- 2010 145 334
- US-A1- 2014 257 206

## Description

L'invention concerne un dispositif d'assistance à l'utilisation d'un dispositif de distribution d'un produit liquide sous forme de gouttes.

Quand on distribue un produit liquide, il est souvent intéressant de connaître la quantité de liquide distribuée. Cela est particulièrement vrai pour l'administration de produits médicaux, pour laquelle la quantité de médicament administrée doit être contrôlée précisément selon la prescription. Une prise insuffisante ou une surdose de médicaments est à éviter pour préserver la santé du patient.

Le document US 2014/0257206 décrit un dispositif de contrôle pour l'instillation de gouttes de liquide ophtalmique comportant un compteur de gouttes par principe optique. Le dispositif est capable de détecter la présence d'une goutte à la sortie d'une bouteille de médicament et ainsi de compter le nombre de gouttes distribuées. Cela permet au dispositif et/ou à l'utilisateur de maîtriser le nombre de gouttes administrées. Cependant, ce dispositif ne donne pas d'information sur la quantité de produit liquide distribué. En effet, on remarque que le volume d'une goutte n'est pas toujours constant. Il dépend de nombreux paramètres variables selon la condition de distribution de chaque goutte.

L'invention a notamment pour but de fournir un dispositif permettant de déterminer la quantité de produit liquide distribué.

À cet effet, l'invention a pour objet un dispositif d'assistance à l'utilisation d'un dispositif de distribution, sur un organe d'un sujet, d'un produit liquide sous forme de gouttes comprenant un réservoir, le dispositif d'assistance comprenant :
- des moyens de solidarisation au dispositif de distribution,
- des moyens optiques destinés à être disposés au voisinage d'un orifice de distribution de produit liquide, configurés pour fournir une information sur la distribution d'une goutte du produit liquide par le dispositif de distribution,
- des moyens de mesure de l'inclinaison configurés pour fournir une information sur l'inclinaison du dispositif de distribution solidarisé au dispositif d'assistance, et
- un système de traitement d'informations sur la distribution d'une goutte et sur l'inclinaison du dispositif de distribution solidarisé au dispositif d'assistance pour fournir une information sur la quantité de produit liquide distribué.

Suite à de nombreux expériences et travaux, on constate que l'inclinaison du dispositif de distribution au moment de la distribution d'une goutte influence le volume de celle-ci, de sorte qu'une mesure de l'inclinaison permet d'estimer le volume d'une goutte à l'issue d'un traitement de cette information. Ainsi, le dispositif d'assistance proposé comprend des moyens de mesure de l'inclinaison du dispositif de distribution auquel il est solidarisé et un système de traitement d'informations, il est donc en mesure de fournir une information pertinente permettant par la suite d'estimer un volume de la goutte distribuée et donc la quantité de produit liquide distribué. De plus, il comporte des moyens optiques permettant de détecter la présence d'une goutte au voisinage de l'orifice de distribution, afin d'obtenir une information sur la distribution d'une goutte du produit liquide par le dispositif de distribution, grâce auxquels le système de traitement d'informations peut alors traiter l'information sur la distribution d'une goutte en lien avec l'inclinaison du dispositif de distribution au moment de la distribution de la goutte. Le dispositif d'assistance ainsi conçu est capable de déterminer une valeur chiffrée de la quantité de produit liquide distribué.

Par ailleurs, à partir de la quantité de produit liquide distribué, le système de traitement d'informations peut avantageusement déduire la quantité de produit liquide restant dans le réservoir du dispositif de distribution. Cette quantité peut être ensuite traduite en nombre théorique de gouttes restantes, en prenant des conditions d'utilisation théoriques du dispositif de distribution. Cette information est suceptible d'intéresser l'utilisateur, lui permettant d'anticiper le moment où le réservoir va être vide et donc d'évaluer son autonomie d'utilisation. Elle peut également être utilisée pour d'autres types d'analyse ou de traitement visant à obtenir des informations supplémentaires.

On notera que le traitement de l'information peut tenir compte d'autres informations mises à disposition du système de traitement d'informations pour estimer la quantité de liquide distribuée, par exemple des informations sur les propriétés physiques ou chimiques du produit liquide, sur la géométrie de la valve, des tableaux de valeurs prédéfinies.

On entend par « optique » toute onde électromagnétique, appartenant au spectre visible ou non. Les moyens optiques sont généralement des moyens capables d'émettre, recevoir et/ou réfléchir une telle onde.

Le dispositif d'assistance peut en outre comporter l'une ou plusieurs des caractéristiques suivantes, prises seules ou en combinaison.
- Le dispositif d'assistance comporte une zone de contact destinée à être en contact avec le réservoir du dispositif de distribution lors de la pression d'activation par l'utilisateur sur le réservoir pour activer la distribution de gouttes, la zone de contact comprenant des moyens pour mesurer la pression d'activation exercée sur la zone de contact pour déclencher les moyens optiques et/ou fournir une information supplémentaire sur la quantité de produit liquide distribué, de préférence une information sur l'intensité de la pression d'activation appliquée et la durée d'application de cette pression d'activation. La pression d'activation exercée sur la zone de contact que mesurent lesdits moyens est une traduction de la pression d'activation exercée par l'utilisateur sur le réservoir, par exemple par un appui direct ou non sur le dispositif d'assistance, appui qui est ensuite transmis au dispositif de distribution ou un appui direct ou non sur le dispositif de distribution par tout moyen adapté. Il se trouve que l'information relative à la pression d'activation exercée sur la zone de contact peut être particulièrement intéressante. En particulier, ne déclencher les moyens optiques qu'une fois que l'utilisateur exerce une certaine pression d'activation permet de réduire leur consommation d'énergie, prolonger leur durée de vie et ne pas générer des informations inutiles en dehors des périodes d'utilisation des dispositifs. Par ailleurs, en plus de l'inclinaison du dispositif de distribution, on constate que la pression d'activation exercée par l'utilisateur est également un paramètre qui influence le volume d'une goutte délivrée. Ainsi, en mesurant cette pression d'activation, le système de traitement d'informations est en mesure de faire une estimation supplémentaire et/ou plus précise de la quantité de produit liquide distribué et de préférence également de la quantité de produit liquide restant dans le réservoir du dispositif de distribution.

On entend généralement par «moyens pour mesurer la pression d'activation » tout moyen permettant de mesurer directement une pression ou une force.
- Le dispositif d'assistance comprend des moyens pour mesurer le poids du dispositif de distribution solidarisé au dispositif d'assistance, configurés pour fournir une information sur la quantité de liquide dans le réservoir. A partir des informations sur la quantité de liquide dans le réservoir avant et après une utilisation, la quantité de produit distribuée peut en être déduite. Cette information peut s'ajouter à celles obtenues à partir d'autres moyens de détection, par exemple les informations sur l'inclinaison du dispositif de distribution, pour obtenir une estimation plus précise du volume de la goutte distribuée et/ou être croisée avec celles-ci pour vérifier les résultats (quantité de produit liquide distribué, quantité de produit liquide restant) à la sortie du système de traitement d'informations. Avantageusement, les moyens pour mesurer le poids comprennent un capteur de poids, par exemple de type capteur de pression (« Force Sensing Resistor » ou FSR en anglais), qui peut être disposé en-dessous du réservoir lorsque le dispositif de distribution est au repos et/ou au-dessus de ce dernier au repos. Un exemple de capteur de poids convenable est le capteur de pression de la marque Flexiforce agissant dans la gamme de forces 0 à 4N. Avantageusement, les moyens pour mesurer le poids comprennent plusieurs capteurs de poids disposés autour du réservoir afin de permettre la mesure du poids du dispositif de distribution solidarisé au dispositif d'assistance, quelle que soit son inclinaison.
- Les moyens optiques comprennent un émetteur et un récepteur d'un signal optique, configurés pour détecter la présence d'une goutte perturbant le signal optique et pour mesurer la durée de cette présence. Le signal optique est par exemple un rayon infrarouge. L'émetteur et le récepteur peuvent être disposés de part et d'autre de l'orifice de distribution lorsque le dispositif d'assistance est solidarisé au dispositif de distribution, de sorte que le signal optique soit traversé par toute goutte distribuée. Eventuellement, l'émetteur et le récepteur sont disposés d'un même coté de l'orifice de distribution lorsque le dispositif d'assistance est solidarisé au dispositif de distribution, le signal optique étant reçu par réflection sur une paroi au moins partiellement réfléchissante. Un exemple de détecteur infra-rouge convenable est un détecteur de la marque OSRAM Opto Semiconductors Inc. ou de la marque Vishay Semiconductor Opto Division. Avantageusement, les moyens optiques comprennent un deuxième émetteur et un deuxième récepteur d'un signal optique, configurés pour valider l'information sur la distribution d'une goutte du produit liquide par le dispositif de distribution. Ces deuxièmes émetteur et récepteur permettent de confirmer que la perturbation du premier signal optique est bien due à la formation puis au détachement d'une goutte de produit liquide.
- Les moyens de mesure de l'inclinaison comprennent un inclinomètre. Ce dernier est par exemple un gyroscope électronique ou un accéléromètre dont les performances sont choisies en fonction du besoin.
- Le système de traitement d'informations comprend des moyens de lecture d'informations portées par le dispositif de distribution. Le système de traitement a alors accès à diverses informations sur le produit liquide contenu dans le dispositif de distribution ou sur le dispositif de distribution lui-même, limitant ou supprimant la nécéssité de paramètrage manuel du dispositif d'assistance pour adapater la distribution aux spécificités du produit à distribuer et/ou au dispositif de distribution. Ces informations sont par exemple le nombre de doses théoriques contenues dans le réservoir, le volume théorique d'une goutte, les propriétés physiques ou chimiques du produit liquide telles que la viscosité, les caractéristiques techniques du dispositif de distribution telles que les dimensions de la valve, ces caractéristiques et propriétés pouvant être basées sur des tableaux de valeurs prédéterminées, des outils de calculs. Une partie de ces informations peuvent être utiles au traitement d'informations fournies par les différents moyens, par exemple les moyens de mesure de l'inclinaison. Le dispositif de distribution peut comporter des supports d'informations sous forme de puces numériques, de bandes magnétiques, de codes barres ou de tout autre type de support dont l'information portée est lisible par voie électronique.
- Le système de traitement d'informations est connecté à un objet extérieur au dispositif d'assistance, par exemple à un serveur, un récepteur, un réseau intranet ou l'internet. Il peut envoyer des informations à distance, et éventuellement en recevoir.
- Le dispositif d'assistance comprend des moyens d'indication des informations fournies par le système de traitement, par exemple des moyens visuels, des moyens sonores et/ou des moyens tactiles. Ces moyens permettent d'indiquer à l'utilisateur des informations sur la bonne délivrance d'une dose, la quantité de produit délivrée et/ou restante et/ou d'autres informations sur le produit liquide (par exemple lues par les moyens de lecture). Les moyens d'affichage peuvent comprendre un écran d'affichage d'informations sous forme alphanumérique et/ou des signaux lumineux, par exemple de couleur ou de forme différentes.
- Le système de traitement d'informations comprend des moyens de stockage d'une valeur variable correspondant à la quantité de produit liquide distribué et/ou à la quantité de produit liquide restant dans le réservoir. La valeur variable est par exemple utilisée pour déduire la quantité de produit liquide distribué et maîtriser la quantité de produit liquide restant dans le réservoir. La valeur variable concernant la quantité de produit liquide distribué peut aussi être utilisée pour controler l'observance du traitement. La valeur variable est mise à jour lors d'une distribution de liquide. Avantageusement, les moyens de stockage sont capables de stocker d'autres valeurs et informations utiles au système de traitement et/ou à l'utilisateur.
- L'invention concerne en outre un kit de distribution d'un produit liquide sous forme de gouttes sur un organe d'un sujet, comprenant un dispositif de distribution du produit liquide et un dispositif d'assistance tel que décrit précédemment. L'organe est par exemple un oeil, une oreille ou la peau. De préférence le dispositif d'assistance et le dispositif de distribution sont des dispositifs distincts, rapportés l'un sur l'autre et amovibles, mais on peut envisager qu'ils soient d'un seul bloc, venus de matière.

L'invention concerne en outre un procédé de détermination de la quantité de produit liquide distribué sous forme de gouttes par un dispositif de distribution de liquide, au moyen d'un dispositif d'assistance tel que décrit ci-dessus, comprenant les étapes suivantes :
- détection d'une goutte,
- estimation du volume de la goutte détectée.

En appliquant le procédé tout le long d'une utilisation du dispositif de distribution et en additionnant le volume estimé de chaque goutte détectée et donc distribuée, on peut obtenir la quantité totale de produit liquide distribué durant cette utilisation.

La détection d'une goutte est réalisée par les moyens optiques du dispositif de distribution. L'ensemble des informations fournies par les moyens optiques et les moyens de mesure de l'inclinaison est traité par le système de traitement d'informations pour estimer le volume de la goutte détectée et en déduire la quantité de produit liquide distribué.

Le procédé de détermination peut en outre comporter l'une ou plusieurs des caractéristiques suivantes, prises seules ou en combinaison.
- Le procédé de détermination comporte une étape de calcul d'une nouvelle valeur de volume résiduel par soustraction du volume de goutte détectée à la valeur précédente de volume résiduel. Le système de traitement d'informations et/ou l'utilisateur est ainsi en connaissance de la quantité de produit liquide restant dans le réservoir après une distribution du produit liquide, cette information n'étant initialement pas accessible depuis l'extérieur du réservoir. On notera que la première valeur correspondant à la « valeur précédente de volume résiduel » est le volume de remplissage du réservoir.

On entend généralement par « volume résiduel », le volume de produit liquide restant dans le réservoir du dispositif de distribution.
- L'étape de détection de la goutte comprend une étape d'identification d'une perturbation d'un signal optique fourni par les moyens optiques et une étape de mesure de la durée de perturbation du signal optique. La perturbation peut être par exemple une absence de signal optique ou un signal optique d'intensité faible en raison de l'absorption du signal optique par la présence de la goutte dans son chemin. La durée de perturbation correspondrait a priori à la durée de la présence de la goutte au voisinage de l'orifice de distribution.
- L'étape d'estimation du volume de la goutte détectée comprend une étape de détermination d'un volume théorique de cette goutte, comprenant de préférence une étape de calcul de ce volume théorique à partir d'informations sur la viscosité du produit liquide et/ou les caractéristiques géométriques de l'orifice de distribution, voire sur d'autres caractéristiques de l'embout de distribution et/ou du dispositif de distribution. Le volume théorique est le volume calculé à partir de données fixes sur le produit liquide ou le dispositif de distribution, ne variant pas en fonction des conditions d'utilisation de ce dernier. Les données fixes peuvent être déterminées par le système de traitement d'informations, par exemple après lecture des informations portées par le dispositif de distribution, ou directement par lecture.
- L'étape d'estimation du volume de la goutte comprend une étape de pondération du volume de goutte détectée, au cours de laquelle l'un au moins des paramètres suivants est pris en compte pour pondérer l'estimation du volume de goutte détectée :
   - l'intensité de la pression d'activation appliquée par l'utilisateur sur le réservoir pour provoquer la formation de la goutte,
   - le profil de variation de cette pression d'activation dans le temps,
   - l'inclinaison du dispositif d'assistance et/ou du dispositif de distribution,
   - la durée d'une perturbation d'un signal fournie par les moyens optiques,
   - la mesure du poids du dispositif de distribution.

En prenant en compte au moins l'un de ces paramètres, on adapte le volume théorique calculé aux conditions d'utilisation du dispositif de distribution, ce qui permet d'obtenir une estimation plus conforme à la réalité et donc une détermination plus précise et fiable de la quantité de produit liquide distribué.
- Le procédé de détermination comprend une étape de mesure du poids du dispositif de distribution pour valider la valeur calculée de volume résiduel. En connaissance de la valeur du volume résiduel par mesure du poids, il est possible d'en déduire la quantité de produit liquide distribué. Ainsi, la mesure du poids permet de valider également le volume estimé de la goutte détectée. L'étape de mesure du poids peut être déclenchée de manière conditionnelle en fonction de l'inclinaison détectée, notamment en fonction du maintien d'une certaine inclinaison du dispositif de distribution pendant une durée prédéterminée.
- Le procédé de détermination comprend une étape de test sur la présence d'un capuchon de protection sur le dispositif de distribution. Le fait de remettre le capuchon est avantageux pour s'assurer de la protection du dispositif de distribution ou pour améliorer l'hygiène et la fonction anti-microbienne en cas de quantité résiduelle de produit liquide dans l'orifice de distribution. Ce test permet de savoir si le capuchon a bien été remis en place à la fin de l'utilisation et éventuellement prévenir l'utilisateur si ce n'est pas le cas. Par ailleurs, lorsque le procédé de détermination comprend une étape de mesure du poids du dispositif de distribution, la présence ou non du capuchon influence la valeur du poids mesuré et doit donc être prise en compte dans la détermination du volume résiduel.
- L'étape de détection de goutte est déclenchée par la détection d'une pression d'activation par un utilisateur sur le réservoir du dispositif de distribution pour activer la distribution de gouttes. En effet, pour activer la distribution de gouttes, l'utilisateur doit exercer une pression d'activation sur le réservoir. La détection de cette pression d'activation marque le début d'une utilisation et est un moment adapté pour déclencher l'étape de détection de goutte sans risque d'omettre la détection d'une goutte. La détection de cette pression d'activation peut être :
   - directe, par mesure de la pression appliquée par l'utilisateur sur la zone d'appui pour activer le dispositif de distribution, ou
   - indirecte, par exemple par mesure de la pression exercée sur la zone de contact destinée à être en contact avec le réservoir du dispositif de distribution lors de la pression d'activation par l'utilisateur sur le réservoir ou sur une autre zone d'appui.

Nous allons maintenant présenter des modes de réalisation particuliers de l'invention, donnés à titre d'exemples non limitatifs et à l'appui des figures annexées sur lesquelles :
- les figures 1A et 1B sont des vues en perspective d'un dispositif d'assistance selon un mode de réalisation, la figure 1A représentant le dispositif d'assistance seul et la figure 1B représentant la partie supérieure de l'ensemble du dispositif d'assistance et d'un dispositif de distribution solidarisé au dispositif d'assistance, le dispositif d'assistance étant en position ouverte et le dispositif de distribution étant pourvu d'un capuchon de protection de l'orifice de distribution,
- les figures 2A, 2B à 4 sont des vues schématiques en coupe longitudinale, et en perspective pour la figure 3, de différentes parties de l'ensemble de la figure 1B,
- la figure 5 est une vue schématique d'une partie d'un ensemble d'un dispositif d'assistance et d'un dispositif de distribution selon un autre mode de réalisation,
- la figure 6 est une vue en perspective de haut du dispositif d'assistance de la figure 1A, sans la partie supérieure,
- la figure 7 est une vue en coupe longitudinale de la partie supérieure du dispositif de distribution de la figure 1B,
- la figure 8 est une vue en perspective de différents orifices de distribution de produit liquide sous forme de gouttes,
- la figure 9 est un graphique montrant l'évolution du volume d'une goutte en fonction de l'inclinaison du dispositif de distribution pendant une distribution de produit liquide,
- la figure 10 est un graphe montrant les étapes d'un procédé de détermination de la quantité de produit liquide distribué par le dispositif de distribution de liquide de la figure 1B,
- la figure 11 est un ensemble d'un graphique montrant des informations traitées par le dispositif d'assistance de la figure 1B et de trois vues en coupe longitudinale de la partie supérieure du dispositif de distribution de la figure 1B à différentes étapes de la distribution d'une goutte,
- les figures 12 et 13 sont deux graphes montrant les étapes d'un procédé de détermination de la quantité de produit liquide distribué selon deux autres modes de réalisation.

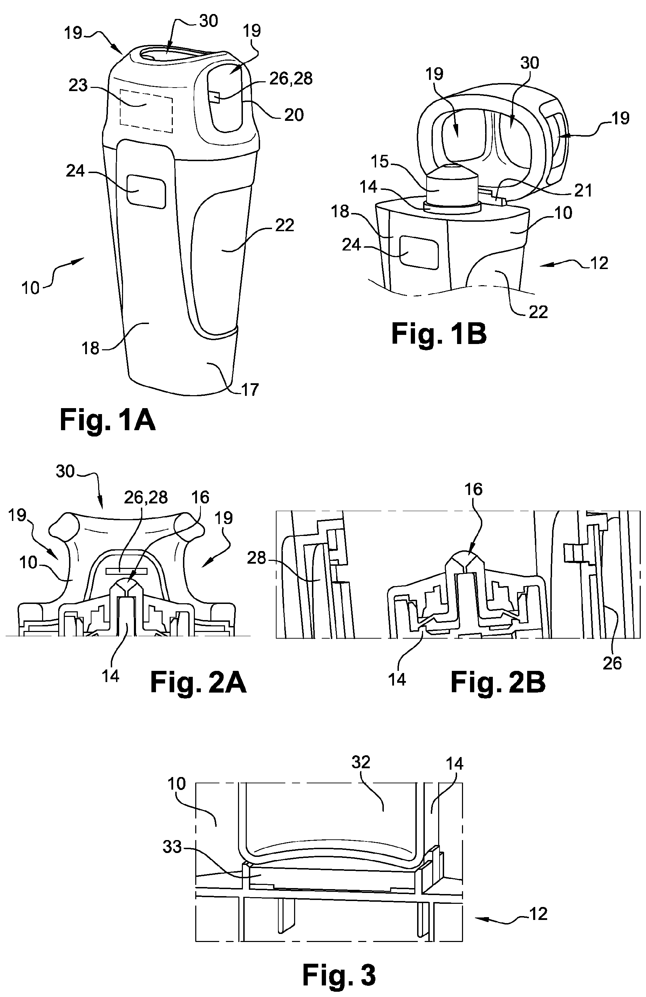

La figure 1A illustre un dispositif d'assistance 10 à l'utilisation d'un dispositif de distribution d'un produit liquide sous forme de gouttes et, la figure 1B, un kit de distribution 12 comprenant le dispositif d'assistance 10 et un dispositif de distribution 14 disposé à l'intérieur du dispositif d'assistance 10. Le dispositif de distribution 14 comprend ici un réservoir 32 (visible sur la figure 3) et un embout de distribution pourvu d'un orifice de distribution 16 des gouttes (visible sur la figure 2B) protégé par un capuchon 15, par exemple vissé sur l'embout de distribution. Le dispositif d'assistance 10 comprend des moyens de solidarisation 17 au dispositif de distribution 14 de sorte que le kit de distribution 12 forme un ensemble solidaire. Les moyens de solidarisation 17 peuvent comprendre par exemple un encliquetage du réservoir 32 dans le dispositif d'assistance 10. Dans une variante parmi d'autres, l'embout de distribution pourvu de l'orifice de distribution 16 pourrait faire partie du dispositif d'assistance 10, rapporté sur le réservoir 32 du dispositif de distribution 14 au moment de la solidarisation des deux dispositifs d'assistance 10 et de distribution 14.

Le dispositif d'assistance 10 comprend un corps principal 18 dans lequel on place le dispositif de distribution 14, et une structure d'appui 20 contre la peau de l'utilisateur lors de la distribution de gouttes dans un organe cible, par exemple un oeil. La structure d'appui 20 est montée amovible sur le corps principal 18 entre une position ouverte d'insertion du dispositif de distribution 14 et une position fermée d'utilisation, par exemple au moyen d'une charnière 21. La structure d'appui 20 peut être conçue de manière assez souple pour assurer le confort de l'appui contre la peau de l'utilisateur et s'adapter aux reliefs au voisinage de l'organe cible, et/ou assez rigide pour assurer la fonction de support à l'appui et imposer une distance prédéterminée entre l'orifice de distribution 16 et l'organe cible. La structure d'appui 20 comprend un orifice axial 30 destiné à autoriser le passage de gouttes de produit liquide de l'orifice de distribution 16 vers l'organe de l'utilisateur. La structure d'appui 20 comprend, de manière facultative, des évidements 19 sur deux côtés opposés et à son extrémité, notamment pour éviter que l'œil de l'utilisateur soit dans le noir lorsque le dispositif d'assistance 10 est appliqué contre la peau de l'utilisateur autour de son oeil. La structure d'appui 20 peut avoir un contour fermé ou non, par exemple un contour en C. Le contour en C permet par exemple à l'utilisateur de tirer la paupière basse au travers de l'ouverture du C afin d'ouvrir davantage son œil et garantir que la goutte atteigne l'œil.

Le dispositif d'assistance 10 comporte également une zone d'appui 22 destinée ici à la fois à la préhension et à permettre l'appui de l'utilisateur pour distribuer le produit liquide. La zone d'appui 22 est ici disposée sur deux côtés opposés du corps principal 18. On peut envisager, dans un autre mode de réalisation, une zone d'appui unique disposée sur un seul coté du corps principal 18. Une pression d'activation exercée sur la zone d'appui 22 est transmise au réservoir 32 du dispositif de distribution 14, en particulier au niveau d'une zone de contact 29 entre le réservoir et le dispositif d'assistance 10. La zone d'appui 22 peut être réalisée en un matériau différent, en particulier plus souple, que celui du reste du corps principal 18. Elle peut également comprendre des reliefs qui facilitent la préhension par l'utilisateur. Par ailleurs, grâce à la présence de la zone d'appui 22, le dispositif d'assistance 10 augmente la surface de préhension de l'utilisateur et de pression d'activation sur le réservoir 32 par rapport à celle du dispositif de distribution 14 seul, ce qui est particulièrement avantageux pour des utilisateurs atteints de maladies neuromusculaires.

Le dispositif d'assistance 10 comprend en outre des moyens de mesure de l'inclinaison configurés pour fournir une information sur l'inclinaison du dispositif de distribution 14 solidarisé au dispositif d'assistance 10. Dans un exemple, les moyens de mesure de l'inclinaison comprennent un inclinomètre tel qu'un gyroscope électronique ou un accéléromètre. L'inclinomètre est de préférence placé dans le corps principal 18, par exemple dans une zone destinée à être placée au voisinage de l'embout de distribution.

Le dispositif d'assistance 10 comprend un système de traitement d'informations 23, notamment de traitement des informations sur la distribution d'une goutte et sur l'inclinaison du dispositif de distribution 14 solidarisé au dispositif d'assistance 10 pour fournir une information sur la quantité de produit liquide distribuée. Le système de traitement d'informations 23 est un système comprenant un ensemble de composants (mécaniques, électroniques, chimiques, photoniques et/ou biologiques) capable de traiter automatiquement des informations. Il comprend par exemple un circuit imprimé de type PCB (pour « printed circuit board »), un ensemble de transistors et/ou un calculateur.

Avantageusement, le dispositif d'assistance 10 comporte une source d'énergie intégrée, par exemple une batterie portable, pour alimenter les différents composants, en particulier les moyens de mesure de l'inclinaison et le système de traitement d'informations 23. Alternativement, il est alimenté en énergie par une source d'alimentation externe.

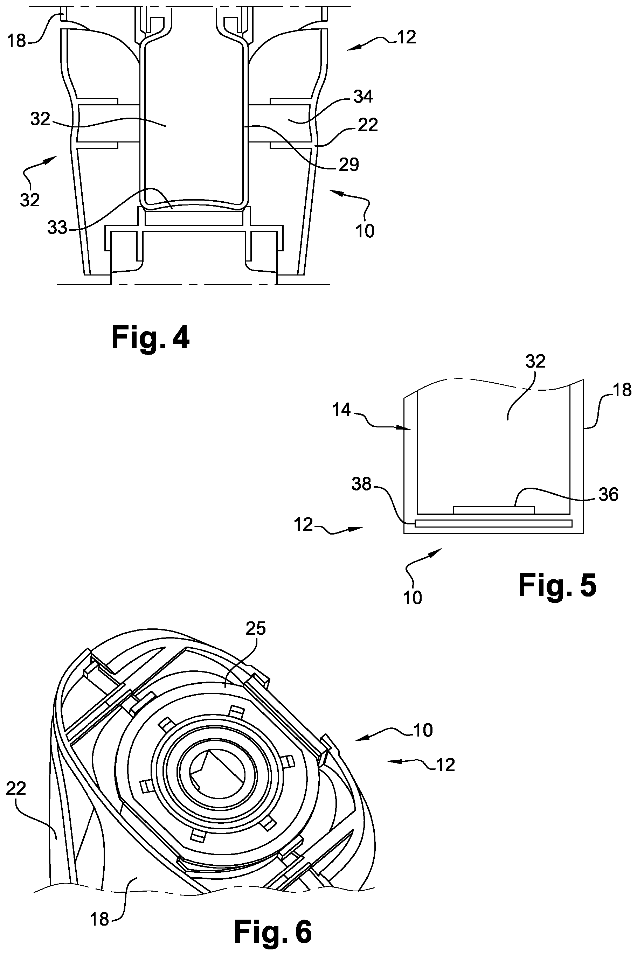

Le dispositif d'assistance 10 comprend, de manière avantageuse, des moyens d'indication des informations fournies par le système de traitement 23, par exemple des moyens visuels 24, 25, des moyens sonores et/ou des moyens tactiles. Dans le mode de réalisation de la figure 1A et 1B, il dispose d'un écran d'affichage 24 d'informations sous forme alphanumérique. Comme on peut le voir sur la figure 6, le dispositif d'assistance 10 comprend en outre ou de façon alternative des diodes électroluminescentes 25 autour de l'orifice de distribution 16 permettant de fournir un signal lumineux pour indiquer par exemple une bonne inclinaison ou une bonne force de pression.

Comme illustré dans la figure 2B, le dispositif d'assistance 10 comprend des moyens optiques 26, 28 destinés à être disposés au voisinage de l'orifice de distribution 16, et configurés pour fournir une information sur la distribution d'une goutte du produit liquide par le dispositif de distribution 14. Les moyens optiques 26, 28 comprennent ici un émetteur 26 et un récepteur 28 d'un signal optique 40, configurés pour détecter la présence d'une goutte perturbant le signal optique 40 et pour mesurer la durée de cette présence. L'émetteur 26 comprend par exemple des diodes émettrices de rayons infrarouges et le récepteur 28 comprend par exemple des photo-transistors aptes à détecter des rayons infrarouges. L'émetteur 26 et le récepteur 28 détectent la présence d'une goutte traversant le signal optique 40 lorsque les rayons optiques sont perturbés, par exemple par une variation de l'intensité des rayons. L'émetteur 26 et le récepteur 28 sont de préférence situés à une distance entre 1 et 3 mm, préférentiellement 2 mm, de l'orifice de distribution 16.

Avec un récepteur de dimensions limitées, il se peut que, lorsque le dispositif d'assistance est incliné, le passage d'une goutte ne soit pas détecté par ce récepteur. Pour pallier ce problème, le récepteur peut présenter avantageusement une zone de réception s'étendant axialement et/ou circonférentiellement et garantissant la détection du passage d'une goutte même lorsque le dispsoitif d'assitance est incliné.

Comme on peut le voir sur la figure 3, le dispositif d'assistance 10 comprend des moyens pour mesurer le poids 33 du dispositif de distribution 14 solidarisé au dispositif d'assistance 10, configurés pour fournir une information sur la quantité de liquide dans le réservoir 32. Les moyens pour mesurer le poids comprennent un capteur de poids 33, par exemple de type capteur de pression (« Force Sensing Resistor » ou FSR en anglais), disposé en dessous du réservoir 32 pour peser le dispositif de distribution 14 et en déduire le poids, et donc le volume, de la quantité de liquide restant dans le réservoir 32. Dans une variante non représentée, le capteur de poids est situé au-dessus du réservoir. Dans une autre variante non représentée, le dispositif d'assistance présente plusieurs capteurs de poids autour du réservoir afin de pouvoir mesurer le poids du dispositif de distribution solidarisé au dispositif d'assistance quelle que soit son inclinaison.

Selon un mode de réalisation non représenté, le dispositif d'assistance peut comporter des moyens de détection du dispositif de distribution solidarisé au dispositif d'assistance, par exemple un capteur de pression ou encore un capteur optique, le système de traitement d'informations étant apte à confirmer à l'utilisateur la présence d'un dispositif de distribution et/ou à l'informer de l'absence de dispositif de distribution.

Le dispositif d'assistance 10 et le dispositif de distribution 14 peuvent comporter des moyens de blocage permettant d'empêcher la rotation du dispositif de distribution 14 par rapport au dispositif d'assistance 10. Les moyens de blocage sont particulièrement utiles lorsque le réservoir 32 est cylindrique. Les moyens de blocage peuvent comporter des formes d'engagement complémentaires prévues respectivement sur le dispositif d'assistance 10 et sur le dispositif de distribution 14, par exemple un ergot logé dans une encoche. La rotation étant ainsi empêchée, l'utilisateur peut revisser le capuchon sur l'embout de distribution sans que celui-ci ne tourne librement. De plus ces moyens de blocage, au contraire d'un ajustement serré entre le dispositif d'assistance et le dispositif de distribution, n'empêchent pas la mesure du poids du dispositif de distribution 14.

Comme on peut le voir sur la figure 4, la zone de contact 29 entre le réservoir 32 et la zone d'appui 22 du dispositif d'assistance 10 comprend des moyens pour mesurer la pression d'activation exercée sur la zone de contact 29 pour déclencher les moyens optiques 26, 28 et/ou fournir une information supplémentaire sur la quantité de produit liquide distribué. Les moyens pour mesurer la pression d'activation 34 peuvent fournir une information sur l'intensité de la pression d'activation appliquée sur la zone de contact 29 et la durée d'application de cette pression d'activation. La zone de contact 29 est située sur une surface interne de la paroi du corps principal 18 portant la zone d'appui 22. Les moyens pour mesurer la pression d'activation comprennent par exemple un capteur de pression 34 de type FSR, placé en contact du réservoir 32.

Comme on peut le voir sur le mode de réalisation de la figure 5, applicable également au mode de réalisation de la figure 1A, le dispositif d'assistance 10 comprend des moyens de lecture d'informations 38 portées par le dispositif de distribution 14. Le dispositif de distribution 14 comprend un support d'informations 36 lisible par voie électronique. Ce support d'informations est par exemple une radio-étiquette 36 (de type tag RFID ou « radio frequency identification » en anglais) collée en-dessous du réservoir 32. La radio-étiquette 36 comprend des informations telles que, dans cet exemple, le volume de remplissage du réservoir 32 (et/ou traduit en nombre de gouttes théorique), le diamètre de l'orifice de distribution 16, la viscosité du produit liquide, le dosage du produit liquide, la date d'expiration/fabrication. Les moyens de lecture comprennent dans cet exemple une antenne 38 apte à lire la radio-étiquette 36 pour en extraire les informations nécessaires au traitement des informations. L'étiquette peut également être collée sur le coté du réservoir ou à tout autre emplacement adapté.

Dans une variante, le système de traitement d'informations 23 est connecté à un objet extérieur au dispositif d'assistance 10, par exemple à un serveur, un récepteur, un réseau intranet ou l'internet.

De façon avantageuse, le système de traitement d'informations 23 comprend des moyens de stockage 39 d'une valeur variable sur la quantité de produit liquide restant dans le réservoir 32.

On décrit maintenant le procédé de détermination de la quantité de produit liquide distribué sous forme de gouttes par le dispositif de distribution 14 de liquide, au moyen du dispositif d'assistance 10.

Pour déterminer la quantité de produit liquide distribué, on détecte d'abord la présence d'une goutte au voisinage de l'orifice de distribution 16 grâce aux moyens optiques 26, 28, ce qui déclenche la mesure de l'inclinaison du dispositif d'assistance 10. Les informations détectées par les moyens optiques 26, 28 et les moyens de mesure de l'inclinaison sont ensuite envoyées au système de traitement d'informations 23 qui les analyse pour fournir une estimation du volume de la goutte détectée. Dans un autre mode de réalisation, on détecte d'abord la variation de l'inclinaison du dispositif d'assistance 10 avant de déclencher l'allumage des moyens optiques 26, 28. Connaissant l'inclinaison du dispositif d'assistance 10 et la géométrie des dispositifs d'assistance 10 et de distribution 14, on connait l'inclinaison du dispositif de distribution 14. L'application de ce procédé de détermination de la présence d'une goutte durant l'utilisation du dispositif de distribution 14 permet d'obtenir une valeur représentative de la quantité de produit liquide distribué goutte par goutte. A partir de cette valeur, on peut calculer une nouvelle valeur de volume résiduel par soustraction du volume estimé de la goutte détectée ou de la quantité de produit liquide distribué à la valeur précédente de volume résiduel de produit liquide restant dans le réservoir 32. Lors d'une première utilisation, la valeur précédente du volume résiduel est le volume de remplissage du réservoir 32. La valeur de volume résiduel est une variable mise à jour après la distribution d'une goutte ou après une utilisation (plusieurs gouttes distribuées) et peut être stockée dans les moyens de stockage du dispositif d'assistance 10.

De préférence, on détecte la présence d'une goutte en identifiant une perturbation du signal optique 40 fourni par les moyens optiques 26, 28, perturbation générée par la présence d'une goutte dans le signal optique 40 entre l'émetteur 26 et le récepteur 28, et plus préférentiellement, en mesurant aussi la durée de perturbation du signal optique 40.

L'estimation du volume de la goutte détectée peut se faire en deux étapes. Une première étape consiste à déterminer un volume théorique de la goutte, par exemple à partir d'informations sur la viscosité du produit liquide et/ou les caractéristiques géométriques de l'orifice de distribution 16, voire sur d'autres caractéristiques de l'embout de distribution. Une méthode de détermination du volume théorique d'une goutte consiste à prendre comme valeur le volume d'une goutte lorsque le dispositif de distribution 14 est incliné d'un angle α (alpha) de 90° par rapport à l'horizontale, en tenant compte du diamètre de l'orifice de distribution 16 et de la viscosité du produit liquide, deux paramètres fixes influençant de manière importante le volume d'une goutte distribuée. On entend par paramètre fixe un paramètre qui ne dépend pas des conditions d'utilisation du dispositif d'assitance 10, telles que l'inclinaison, la pression d'activation, la vitesse d'appui.... Ces informations sont par exemple lues par le dispositif d'assistance 14 sur une radio-étiquette 36 du dispositif de distribution 14. On comprend par ailleurs que l'angle d'inclinaison α correspond à l'angle formé par l'axe du dispositif de distribution 14, correspondant à l'axe de l'orifice de distribution 16, par rapport à une direction horizontale, en référence à la direction de la gravité qui définit une direction verticale.

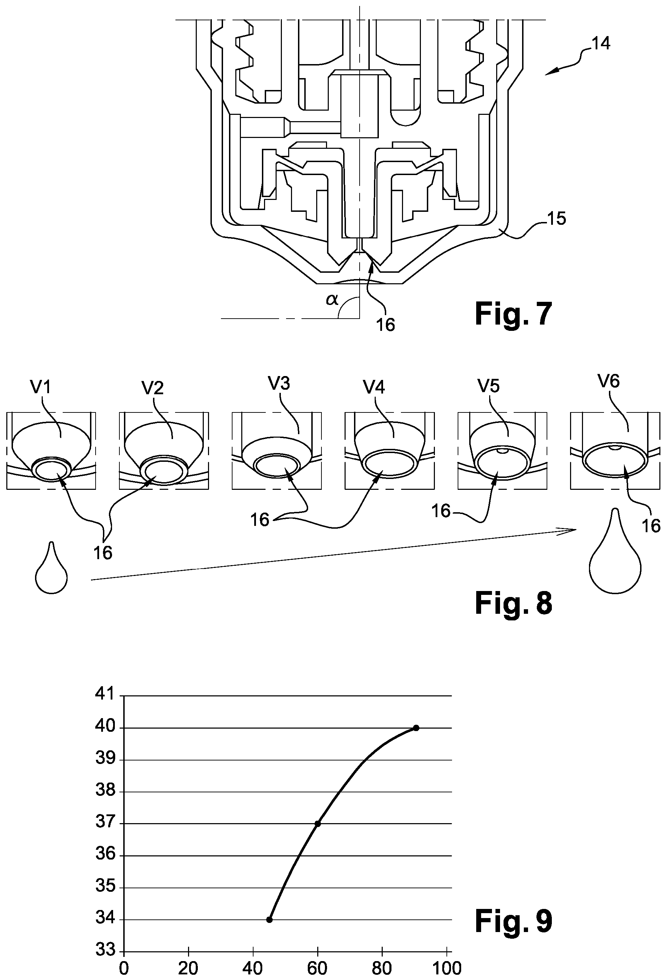

Comme le montre la figure 8 qui illustre six valves de distribution V1 à V6 de dispositif de distribution 14 ayant différentes formes et dimensions, on constate que, de manière générale, le volume d'une goutte croît avec le diamètre de l'orifice de distribution 16. Le tableau ci-dessous est un exemple de correspondance entre le diamètre de l'orifice de distribution 16 et le volume théorique de la goutte. Les résultats ont été obtenus avec de l'eau, en inclinant le dispositif de distribution 14 d'un angle α de 90°.

| | | | | | | |
|---|---|---|---|---|---|---|
| Valve et dimension de l'orifice (en mm) | V1 : 1,6 | V2 : 2,0 | V3 : 2,4 | V4 : 2,7 | V5 : 3,0 | V6 : 3,6 |
| Volume théorique de la goutte (en µL) | 28 | 33 | 40 | 43 | 46 | 53 |

Par ailleurs, on constate que la viscosité du produit liquide influence le volume d'une goutte distribué, ceci même sans prendre en compte des paramètres variables en fonction des conditions d'utilisation du dispositif de distribution 14. En effet, on note que le volume d'une goutte dans des conditions théoriques d'utilisation (dispositif de distribution 14 incliné à un angle α de 90° par rapport à l'horizontale avec la valve V3) augmente avec la viscosité du produit liquide. Le tableau ci-dessous est un exemple de correspondance entre la viscosité du produit liquide et le volume théorique de la goutte.

| | | | | |
|---|---|---|---|---|
| Viscosité (en Cp) | 0 | 50 | 200 | 1000 |
| Volume théorique de la goutte en µL) | 40 | 41 | 42 | 50 |

Une deuxième étape consiste à pondérer le volume théorique en prenant en compte au moins l'un des paramètres suivants :
- l'intensité de la pression d'activation appliquée par l'utilisateur sur le réservoir 32 pour provoquer la formation de la goutte,
- le profil de variation de cette pression d'activation dans le temps,
- l'inclinaison du dispositif d'assistance 10 et du dispositif de distribution 14,
- la durée d'une perturbation d'un signal optique 40 fourni par les moyens optiques,
- la mesure du poids du dispositif de distribution 14.

Une méthode de pondération du volume théorique consiste par exemple à multiplier sa valeur par un coefficient A1 lié à l'inclinaison du dispositif d'assistance 10 et du dispositif de distribution 14 fournie par les moyens de mesure de l'inclinaison et par un coefficient A2 lié à la durée d'une perturbation d'un signal optique 40 fourni par les moyens optiques 26, 28 et à la viscosité du produit liquide contenu dans le réservoir 32.

En effet, on constate que l'inclinaison du dispositif de distribution 14 durant la distribution d'une goutte influence le volume de cette dernière. Comme le montre le graphique de la figure 9, le volume de la goutte distribuée (en ordonnée) augmente avec l'angle d'inclinaison α du dispositif de distribution 14 (en abscisse) mesuré par rapport à l'horizontale. Par exemple, on mesure un volume de 34µl à un angle d'inclinaison α de 45°, un volume de 37µl à un angle d'inclinaison α de 60° et un volume de 40µl à un angle d'inclinaison α de 90°, le produit liquide utilisé étant l'eau et la valve utilisée étant la valve V3 utilisée comme référence. Après avoir reçu la valeur de l'inclinaison fournie par les moyens de mesure de l'inclinaison, le système de traitement d'informations 23 calcule le coefficient A1 en divisant cette valeur de l'inclinaison par le volume théorique d'une goutte d'eau lorsque le dispositif de distribution 14 est incliné à un angle α de 90°par rapport à l'horizontal en prenant la valve V3, soit 40µl.

Par ailleurs, on constate que le volume de la goutte distribuée peut varier avec la viscosité du produit liquide en fonction de la vitesse de formation de la goutte. Il est donc également intéressant de pondérer le volume théorique d'une goutte par un coefficient A2 tenant compte de la viscosité du produit liquide et de la vitesse de formation de la goutte. Cette vitesse de formation de la goutte est obtenue avantageusement à l'aide de la durée de perturbation du signal optique 40 et/ou de l'intensité et la durée de la pression d'activation exercée pour distribuer une goutte. Le tableau ci-dessous donne un exemple de correspondance entre un ensemble formé par la viscosité du produit liquide et la vitesse de formation de la goutte, et le coefficient A2.

| Vitesse (en s) | Viscosité (en Cp) | | | | |
|---|---|---|---|---|---|
| | 0 | 50 | 200 | 1000 | |
| <1s | 0,75 | 0,80 | 1,07 | 1,20 | A2 |
| >2s | 0,75 | 1,00 | 1,00 | 1,00 | |

Dans une variante, le volume théorique d'une goutte peut également être pondéré par un coefficient lié à l'intensité de la pression d'activation appliquée par l'utilisateur sur le réservoir 32 pour provoquer la formation de la goutte et/ou le profil de variation de cette pression d'activation dans le temps.

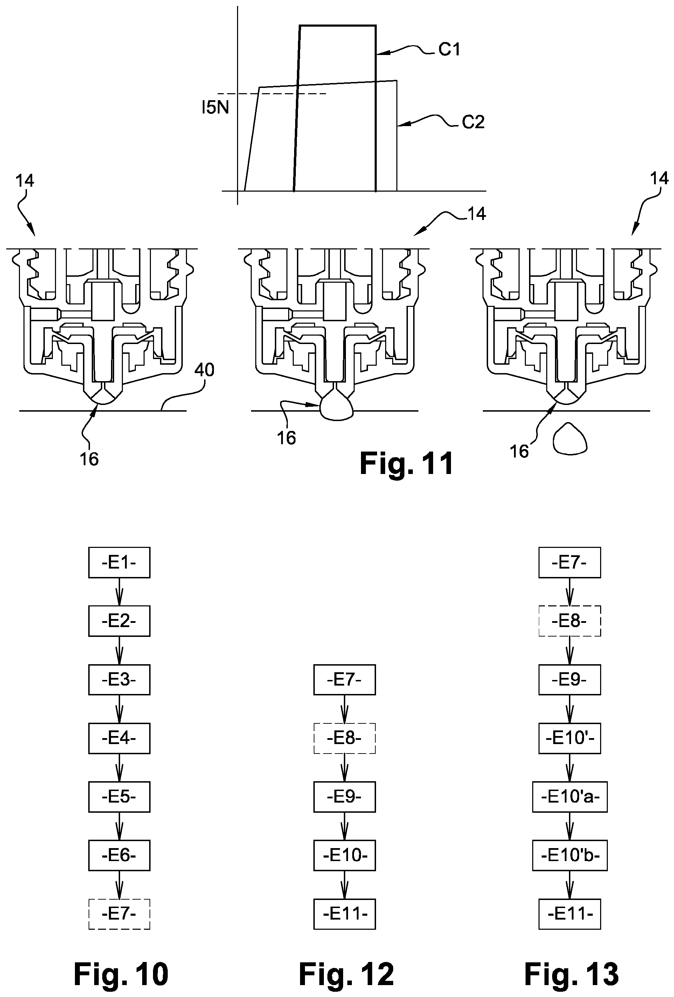

Un exemple des étapes d'un procédé de détermination est décrit à la figure 10. Le procédé débute par une première étape E1 de détection d'une pression d'activation exercée par l'utilisateur sur la zone d'appui 22, pression qui est transmise au réservoir 32 du dispositif de distribution 14 via la zone de contact 29 entre ce dernier et le dispositif d'assistance 10 où est disposé un capteur de pression d'activation. Lorsque la pression d'activation détectée dépasse un seuil prédéterminé, par exemple 15N, les moyens optiques 26, 28 et les moyens de mesure de l'inclinaison sont alors activés pour la détection d'une goutte et la mesure de l'inclinaison du dispositif de distribution 14 dans une étape E2. Les moyens optiques 26, 28 surveillent la zone de formation de la goutte au voisinage de l'orifice de distribution 16 jusqu'à ce qu'ils détectent la présence d'une goutte (étape E3). Un compteur de temps est lancé à l'étape E4 au moment où la goutte est détectée. Le temps est compté tant que la goutte ne quitte pas la zone de formation surveillée par les moyens optiques, *i.e.* ne se détache pas de l'orifice de distribution 16. Une fois que la goutte quitte la zone de formation pour être distribuée (étape E5), le signal optique 40 des moyens optiques 26, 28 est modifié, plus précisément le signal optique 40 n'est plus perturbé, et le compteur de temps s'arrête pour fournir une durée de formation de la goutte au système de traitement d'informations 23 qui incrémente d'une unité le nombre de gouttes distribuées (étape E6). Les moyens de mesure de l'inclinaison fournissent les mesures de l'inclinaison au système de traitement d'informations 23 pour l'estimation du volume de la goutte (étape E6). Dans une étape E7 facultative, le volume résiduel peut en être déduit, ainsi que le nombre de gouttes restant dans le réservoir 32. Ce procédé peut se répéter plusieurs fois tant qu'une pression d'activation dépassant le seuil prédéterminé est détectée et/ou que les moyens optiques 26, 28 détectent la présence d'une goutte.

La figure 11 représente un graphique ayant pour abscisse le temps et pour ordonnée l'intensité de la pression d'activation ou de la perturbation du signal optique 40. Le graphique est un exemple de représentation des informations que reçoit le système de traitement d'informations 23 des moyens optiques 26, 28 (courbe C1) et des moyens pour mesurer la pression d'activation 34 (courbe C2) exercée par l'utilisateur durant la distribution d'une goutte. Lorsque la pression d'activation mesurée dépasse un seuil prédéterminé, par exemple 15N, les moyens optiques 26, 28, ici l'émetteur 26 et le récepteur 28 d'un signal optique 40, sont activés. Lorsqu'une goutte se forme entre l'émetteur 26 et le récepteur 28, le signal optique 40 est perturbé, ce qui génère une intensité élevée de la perturbation. Cette perturbation disparaît une fois que la goutte se détache de l'orifice de distribution 16.

Un autre exemple de procédé de détermination est représenté à la figure 12, permettant de vérifier la valeur du volume résiduel calculée à l'étape E7 de l'exemple de la figure 10 par une mesure du poids du dispositif de distribution 14. Les étapes E1 à E7 sont similaires à celles de l'exemple de la figure 10 et ne sont pas reprises. A la suite du calcul du volume résiduel à l'étape E7, les moyens de mesure de l'inclinaison continuent à fournir des données au système de traitement d'informations 23. Ce dernier effectue une étape E8 de test de la position verticale. Une fois que le dispositif de distribution 14 et/ou d'assistance 10 revient à sa position de repos, le système de traitement d'informations 23 valide la position verticale et une étape E9 de mesure du poids du dispositif de distribution 14 est exécutée par des moyens adaptés du dispositif d'assistance 10. Dans une variante, l'étape E8 n'est pas effectuée, c'est-à-dire qu'on mesure le poids du dispositif de distribution 14 quel que soit l'inclinaison du dispositif de distribution 14 et/ou d'assistance 10, par exemple après une variation importante de l'inclinaison du dispositif de distribution 14 après une distribution de gouttes. Le poids mesuré à l'étape E9 est différent en fonction de la présence ou non du capuchon 15 du dispositif de distribution 14. On effectue ainsi une étape E10 de vérification de la remise en place du capuchon 15. Cette vérification est soit faite manuellement par l'utilisateur qui fournit cette information au dispositif d'assistance 10, soit de manière plus avantageuse effectuée par des moyens de détection du capuchon 15 situés sur le dispositif d'assistance 10. Les moyens de détection du capuchon 15 peuvent comprendre par exemple un capteur de contact mécanique ou par la pression d'activation, ou plus avantageusement les moyens optiques. En effet, en fonction de la nature de la perturbation du signal optique 40, le système de traitement d'informations 23 et/ou les moyens optiques 26, 28 peuvent différencier la présence d'une goutte de liquide ou d'un capuchon 15. En fonction du résultat obtenu à l'étape E10, le système de traitement d'informations 23 calcule, dans une étape E11, la quantité de liquide restant dans le réservoir 32 à partir du poids mesuré.

La figure 13 représente une variante du mode de réalisation de la figure 12. Dans cette variante, l'étape E10 de vérification de la remise en place du capuchon 15 est effectuée uniquement par le système de traitement d'informations 23 avec des données calculées, préconfigurées et/ou lues sur le dispositif de distribution 14. Cette étape E10' comprend deux sous-étapes successives E10'a et E10'b. Dans l'étape E10'a, le système de traitement d'informations 23 estime le poids du dispositif de distribution 14 avec (respectivement sans) capuchon 15 en additionnant le poids du volume résiduel (obtenu à l'étape E6) et le poids à vide du dispositif de distribution 14 avec (respectivement sans) capuchon 15. Dans l'étape E10'b, le système de traitement d'informations 23 compare le poids mesuré avec le poids estimé du dispositif de distribution 14 avec (respectivement sans) capuchon 15. Si le poids mesuré est très inférieur au poids estimé du dispositif de distribution 14 avec capuchon 15 et/ou environ égal au poids estimé du dispositif de distribution 14 sans capuchon 15, alors le système de traitement d'informations 23 en déduit que le capuchon 15 n'a pas été remis sur le dispositif de distirbution 14. Si le poids mesuré est très supérieur au poids estimé du dispositif de distribution 14 sans capuchon 15 et/ou environ égal au poids estimé du dispositif de distribution 14 avec capuchon 15, alors le système en déduit que le capuchon 15 a bien été remis en place. En fonction du résultat obtenu à l'étape E10', le système de traitement d'informations 23 calcule, à l'étape E11, la quantité de liquide restant dans le réservoir 32 en soustrayant le poids à vide du dispositif de distribution 14 avec ou sans capuchon 15 du poids mesuré.

Selon une variante de réalisation non représentée, la pression d'activation exercée par l'utilisateur sur la zone d'appui est déterminée par l'intermédiaire des moyens pour mesurer le poids du dispositif de distribution solidarisé au dispositif d'assistance. Avec un réservoir souple, la pression d'activation déforme le réservoir radialement dans son diamètre et également longitudinalement dans sa longueur. La déformation dans la longueur peut être mesurée par la balance et l'information sur la pression d'activation déduite. Dans cette variante de réalisation, la zone de contact est distincte de la zone d'appui de l'utilisateur.

L'invention n'est pas limitée aux modes de réalisation présentés et d'autres modes de réalisation apparaîtront clairement à l'homme du métier. Par exemple, il peut faire différentes combinaisons des différents moyens présentés pour obtenir un dispositif d'assistance, un kit de distribution ou un procédé de détermination adapté au besoin.

## Revendications

1. Dispositif d'assistance (10) à l'utilisation d'un dispositif de distribution (14), sur un organe d'un sujet, d'un produit liquide sous forme de gouttes comprenant un réservoir (32), le dispositif d'assistance (10) comprenant :
- des moyens de solidarisation (17) au dispositif de distribution (14),
- des moyens optiques (26, 28) destinés à être disposés au voisinage d'un orifice de distribution (16) du produit liquide, configurés pour fournir une information sur la distribution d'une goutte du produit liquide par le dispositif de distribution (14),
**caractérisé en ce que** le dispositif d'assistance (10) comprend en outre :
- des moyens de mesure de l'inclinaison configurés pour fournir une information sur l'inclinaison du dispositif de distribution (14) solidarisé au dispositif d'assistance (10), et
un système de traitement (23) d'informations sur la distribution d'une goutte et sur l'inclinaison du dispositif de distribution (14) solidarisé au dispositif d'assistance (10) pour fournir une information sur la quantité de produit liquide distribué.

2. Dispositif d'assistance (10) selon la revendication précédente, comportant une zone de contact (29) destinée à être en contact avec le réservoir (32) du dispositif de distribution (14) lors de la pression d'activation par l'utilisateur sur le réservoir (32) pour activer la distribution de gouttes, la zone de contact (29) comprenant des moyens pour mesurer la pression d'activation (34) exercée sur la zone de contact (29) pour déclencher les moyens optiques (26, 28) et/ou fournir une information supplémentaire sur la quantité de produit liquide distribué, de préférence une information sur l'intensité de la pression d'activation appliquée et la durée d'application de cette pression d'activation.

3. Dispositif d'assistance (10) selon l'une quelconque des revendications précédentes, comprenant des moyens pour mesurer le poids (33) du dispositif de distribution (14) solidarisé au dispositif d'assistance (10), configurés pour fournir une information sur la quantité de liquide dans le réservoir (32).

4. Dispositif d'assistance (10) selon l'une quelconque des revendications précédentes, dans lequel les moyens optiques (26, 28) comprennent un émetteur (26) et un récepteur (28) d'un signal optique (40), configurés pour détecter la présence d'une goutte perturbant le signal optique (40) et pour mesurer la durée de cette présence.

5. Dispositif d'assistance (10) selon l'une quelconque des revendications précédentes, dans lequel le système de traitement d'informations (23) comprend des moyens de lecture (38) d'informations portées par le dispositif de distribution (14).

6. Dispositif d'assistance (10) selon l'une quelconque des revendications précédentes, dans lequel le système de traitement d'informations (23) comprend des moyens de stockage (39) d'une valeur variable correspondant à la quantité de produit liquide distribué et/ou à la quantité de produit liquide restant dans le réservoir (32).

7. Kit de distribution (12) d'un produit liquide sous forme de gouttes sur un organe d'un sujet, comprenant un dispositif de distribution (14) du produit liquide et un dispositif d'assistance (10) selon l'une quelconque des revendications précédentes.

8. Procédé de détermination de la quantité de produit liquide distribué sous forme de gouttes par un dispositif de distribution (14) de liquide, au moyen d'un dispositif d'assistance (10) selon l'une quelconque des revendications 1 à 6, comprenant les étapes suivantes :
- détection d'une goutte (E3),
- estimation du volume de la goutte détectée (E6).

9. Procédé de détermination selon la revendication précédente, comprenant une étape (E7) de calcul d'une nouvelle valeur de volume résiduel par soustraction du volume de goutte détectée à la valeur précédente de volume résiduel.

10. Procédé de détermination selon l'une quelconque des revendications 8 à 9, au cours duquel l'étape de détection de la goutte (E3) comprend une étape d'identification d'une perturbation d'un signal optique (40) fourni par les moyens optiques (26, 28) et une étape de mesure de la durée de perturbation du signal optique (40).

11. Procédé de détermination selon l'une quelconque des revendications 8 à 10, dans lequel l'étape d'estimation du volume de la goutte détectée (E6) comprend une étape de détermination d'un volume théorique de cette goutte, comprenant de préférence une étape de calcul de ce volume théorique à partir d'informations sur la viscosité du produit liquide et/ou les caractéristiques géométriques de l'orifice de distribution (16), voire sur d'autres caractéristiques de l'embout de distribution et/ou du dispositif de distribution (14).

12. Procédé de détermination selon la revendication précédente, dans lequel l'étape d'estimation du volume de la goutte (E6) comprend une étape de pondération du volume de goutte détectée, au cours de laquelle l'un au moins des paramètres suivants est pris en compte pour pondérer l'estimation du volume de goutte détectée :
- l'intensité de la pression d'activation appliquée par l'utilisateur sur le réservoir (32) pour provoquer la formation de la goutte,
- le profil de variation de cette pression d'activation dans le temps,
- l'inclinaison du dispositif d'assistance et/ou du dispositif de distribution (14),
- la durée d'une perturbation d'un signal optique (40) fournie par les moyens optiques (26, 28),
- la mesure du poids du dispositif de distribution (14).

13. Procédé de détermination selon l'une quelconque des revendications 9 à 12, comprenant une étape (E9) de mesure du poids du dispositif de distribution (14) pour valider la valeur calculée de volume résiduel.

14. Procédé de détermination selon l'une quelconque des revendications 8 à 13, comprenant une étape (E10, E10') de test sur la présence d'un capuchon (15) de protection sur le dispositif de distribution (14).

15. Procédé de détermination selon l'une quelconque des revendications 8 à 14, au cours duquel l'étape de détection de goutte (E3) est déclenchée par la détection d'une pression d'activation (E1) par un utilisateur sur le réservoir du dispositif de distribution (14) pour activer la distribution de gouttes.

## Patentansprüche

1. Vorrichtung (10) zur Unterstützung der Verwendung einer Abgabevorrichtung (14) für ein flüssiges Produkt in Tropfenform, die ein Reservoir (32) aufweist, auf ein Organ einer Person, wobei die Unterstützungsvorrichtung (10) aufweist:
- Mittel (17) zur festen Verbindung mit der Abgabevorrichtung (14),
- optische Mittel (26, 28), die dazu bestimmt sind, in der Nähe einer Abgabeöffnung (16) des flüssigen Produkts angeordnet zu werden, und die eingerichtet sind, eine Information über die Abgabe eines Tropfens des flüssigen Produkts durch die Abgabevorrichtung (14) zu liefem,
**dadurch gekennzeichnet, dass** die Unterstützungsvorrichtung (10) außerdem aufweist:
- Mittel zur Messung der Neigung, die so konfiguriert sind, dass sie eine Information über die Neigung der Abgabevorrichtung (14) liefern, die fest mit der Unterstützungsvorrichtung (10) verbunden ist, und
ein System (23) zur Verarbeitung von Informationen über die Abgabe eines Tropfens und über die Neigung der Abgabevorrichtung (14), die fest mit der Unterstützungsvorrichtung (10) verbunden ist, um eine Information über die Menge des abgegebenen flüssigen Produkts zu liefern.

2. Unterstützungsvorrichtung (10) nach dem vorhergehenden Anspruch, mit einem Kontaktbereich (29), die dazu bestimmt ist, mit dem Reservoir (32) der Abgabevorrichtung (14) in Kontakt zu sein, wenn der Benutzer Aktivierungsdruck auf das Reservoir (32) ausübt, um die Abgabe von Tropfen zu aktivieren, wobei der Kontaktbereich (29) Mittel zum Messen des Aktivierungsdrucks (34) aufweist, der auf den Kontaktbereich (29) ausgeübt wird, um die optischen Mittel (26, 28) auszulösen und/oder zusätzliche Informationen über die Menge des abgegebenen flüssigen Produkts zu liefern, vorzugsweise Informationen über die Intensität des ausgeübten Aktivierungsdrucks und die Dauer der Anwendung dieses Aktivierungsdrucks.

3. Unterstützungsvorrichtung (10) nach einem der vorhergehenden Ansprüche, mit Mitteln zum Messen des Gewichts (33) der fest mit der Unterstützungsvorrichtung (10) verbundenen Abgabevorrichtung (14), die eingerichtet sind, eine Information über die Menge der Flüssigkeit im Reservoir (32) zu liefern.

4. Unterstützungsvorrichtung (10) nach einem der vorhergehenden Ansprüche, wobei die optischen Mittel (26, 28) einen Sender (26) und einen Empfänger (28) für ein optisches Signal (40) aufweisen, die konfiguriert sind, die Anwesenheit eines das optische Signal (40) störenden Tropfens zu erfassen und die Dauer dieser Anwesenheit zu messen.

5. Unterstützungsvorrichtung (10) nach einem der vorhergehenden Ansprüche, wobei das System (23) zur Verarbeitung von Informationen Mittel zum Lesen (38) von Informationen aufweist, die von der Ausgabevorrichtung (14) getragen werden.

6. Unterstützungsvorrichtung (10) nach einem der vorhergehenden Ansprüche, bei der das System (23) zur Verarbeitung von Informationen (23) Mittel zur Speicherung (39) eines variablen Werts aufweist, der der Menge des abgegebenen flüssigen Produkts und/oder der Menge des im Reservoir (32) verbleibenden flüssigen Produkts entspricht.

7. Set (12) zur Abgabe eines flüssigen Produkts in Form von Tropfen auf ein Organ einer Person, mit einer Abgabevorrichtung (14) für das flüssige Produkt und einer Unterstützungsvorrichtung (10) nach einem der vorhergehenden Ansprüche.

8. Verfahren zum Bestimmen der Menge eines flüssigen Produkts, das von einer Flüssigkeitsabgabevorrichtung (14) in Form von Tropfen abgegeben wird, mithilfe einer Unterstützungsvorrichtung (10) nach einem der Ansprüche 1 bis 6, mit den folgenden Schritten:
- Erfassen eines Tropfens (E3),
- Schätzen des Volumens des erfassten Tropfens (E6).

9. Bestimmungsverfahren nach dem vorhergehenden Anspruch, das einen Schritt (E7) zum Berechnen eines neuen Restvolumenwerts durch Subtraktion des erfassten Tropfenvolumens von dem vorherigen Restvolumenwert umfasst.

10. Bestimmungsverfahren nach einem der Ansprüche 8 bis 9, wobei der Schritt des Erfassens des Tropfens (E3) einen Schritt des Identifizierens einer Störung eines optischen Signals (40), das von den optischen Mitteln (26, 28) geliefert wird, und einen Schritt des Messens der Dauer der Störung des optischen Signals (40) umfasst.

11. Bestimmungsverfahren nach einem der Ansprüche 8 bis 10, bei dem der Schritt zur Schätzung des Volumens des erfassten Tropfens (E6) einen Schritt zur Bestimmung eines theoretischen Volumens dieses Tropfens umfasst, der vorzugsweise einen Schritt zur Berechnung dieses theoretischen Volumens anhand von Informationen über die Viskosität des flüssigen Produkts und/oder die geometrischen Eigenschaften der Abgabeöffnung (16) bzw. über andere Eigenschaften des Abgabeansatzes und/oder der Abgabevorrichtung (14) umfasst.

12. Bestimmungsverfahren nach dem vorhergehenden Anspruch, wobei der Schritt des Schätzens des Tropfenvolumens (E6) einen Schritt des Gewichtens des erfassten Tropfenvolumens umfasst, bei dem mindestens einer der folgenden Parameter berücksichtigt wird, um die Schätzung des erfassten Tropfenvolumens zu gewichten:
- die Stärke des Aktivierungsdrucks, den der Benutzer auf das Reservoir (32) ausübt, um die Bildung des Tropfens zu bewirken,
- das Änderungsprofil dieses Aktivierungsdrucks über die Zeit,
- die Neigung der Unterstützungsvorrichtung und/oder der Abgabevorrichtung (14),
- die Dauer einer Störung eines optischen Signals (40), das von den optischen Mitteln (26, 28) geliefert wird,
- die Messung des Gewichts der Abgabevorrichtung (14).

13. Bestimmungsverfahren nach einem der Ansprüche 9 bis 12, umfassend einen Schritt (E9) des Messens des Gewichts der Abgabevorrichtung (14), um den berechneten Wert des Restvolumens zu validieren.

14. Bestimmungsverfahren nach einem der Ansprüche 8 bis 13, umfassend einen Schritt (E10, E10') des Testens, ob eine Schutzkappe (15) auf der Abgabevorrichtung (14) vorhanden ist.

15. Bestimmungsverfahren nach einem der Ansprüche 8 bis 14, wobei der Schritt des Erfassens eines Tropfens (E3) durch das Erfassen eines Aktivierungsdrucks (E1) durch einen Benutzer auf den Behälter der Abgabevorrichtung (14) ausgelöst wird, um die Abgabe von Tropfen zu aktivieren.

## Claims

1. Assistance device (10) for assisting with the use of a dispensing device (14) for dispensing, on an organ of a subject, a liquid product in the form of drops comprising a reservoir (32), the assistance device (10) comprising:
- means (17) for rigid connection to the dispensing device (14),
- optical means (26, 28) intended to be arranged near a dispensing orifice (16) for dispensing the liquid product, configured to provide an information on the dispensing of a drop of the liquid product by the dispensing device (14),
**characterised in that** the assistance device (10) further comprises:
- means for measuring the inclination, configured to provide information on the inclination of the dispensing device (14) connected to the assistance device (10), and
an information processing system (23) for processing information on the dispensing of a drop and on the inclination of the dispensing device (14) connected to the assistance device (10) to provide an information on the amount of liquid product dispensed.

2. Assistance device (10) according to the preceding claim, comprising a contact area (29) intended to be in contact with the reservoir (32) of the dispensing device (14) when the user exerts an activation pressure on the reservoir (32) to activate the dispensing of drops, the contact area (29) comprising means for measuring the activation pressure (34) exerted on the contact area (29) in order to trigger the optical means (26, 28) and/or provide additional information on the amount of liquid product dispensed, preferably information on the magnitude of the activation pressure applied and the time during which this activation pressure is applied.

3. Assistance device (10) according to any one of the preceding claims, comprising means (33) for measuring the weight of the dispensing device (14) connected to the assistance device (10), configured to provide information on the amount of liquid in the reservoir (32).

4. Assistance device (10) according to any one of the preceding claims, wherein the optical means (26, 28) comprise a transmitter (26) and a receiver (28) of an optical signal (40), configured to detect the presence of a drop disturbing the optical signal (40) and to measure the duration of this presence.

5. Assistance device (10) according to any one of the preceding claims, wherein the information processing system (23) comprises reading means (38) for reading information shown on the dispensing device (14).

6. Assistance device (10) according to any one of the preceding claims, wherein the information processing system (23) comprises storing means (39) for storing a variable value corresponding to the amount of liquid product dispensed and/or the amount of liquid product remaining in the reservoir (32).

7. Kit (12) for dispensing a liquid product in the form of drops on an organ of a subject, comprising a dispensing device (14) for dispensing liquid product and an assistance device (10) according to any one of the preceding claims.

8. Determination method for determining the amount of liquid product dispensed in the form of drops by a liquid dispensing device (14), by means of an assistance device (10) according to any one of claims 1 to 6, comprising the following steps:
- detecting a drop (E3),
- estimating the volume of the drop detected (E6).

9. Determination method according to the preceding claim, comprising a step (E7) of calculating a new value of the residual volume by subtracting the volume of the drop detected from the previous value of the residual volume.

10. Determination method according to claim 8 or 9, during which the step (E3) of detecting the drop comprises a step of identifying a disturbance of an optical signal (40) provided by the optical means (26, 28) and a step of measuring the time during which the optical signal (40) is disturbed.

11. Determination method according to any one of claims 8 to 10, wherein the step (E6) of estimating the volume of the drop detected comprises a step of determining a theoretical volume of this drop, preferably comprising a step of calculating this theoretical volume using information on the viscosity of the liquid product and/or the geometric characteristics of the dispensing orifice (16), or even on other characteristics of the dispensing end piece and/or of the dispensing device (14).

12. Determination method according to the preceding claim, wherein the step (E6) of estimating the volume of the drop comprises a step of weighting the volume of the drop detected, during which at least one of the following parameters is taken into account to weight the estimation of the volume of the drop detected:
- the magnitude of the activation pressure applied by the user on the reservoir (32) to cause the formation of the drop,
- the variation profile of this activation pressure over time,
- the inclination of the assistance device and/or of the dispensing device (14),
- the time during which an optical signal (40) provided by the optical means (26, 28) is disturbed,
- the measurement of the weight of the dispensing device (14).

13. Determination method according to any one of claims 9 to 12, comprising a step (E9) of measuring the weight of the dispensing device (14) to validate the calculated value of residual volume.

14. Determination method according to any one of claims 8 to 13, comprising a step (E10, E10') of testing the presence of a protective cap (15) on the dispensing device (14).

15. Determination method according to any one of claims 8 to 14, during which the step (E3) of detecting a drop is triggered by detection of an activation pressure (E1) by a user on the reservoir of the dispensing device (14) to activate the dispensing of drops.
